(19) Europäisches Patentamt
European Patent Office
Office européen des brevets

(11) EP 4 790 104 A1

(12) # EUROPEAN PATENT APPLICATION

published in accordance with Art. 153(4) EPC

(43) Date of publication:
**12.08.2026 Bulletin 2026/33**

(21) Application number: **24874406.2**

(22) Date of filing: **29.08.2024**

(51) International Patent Classification (IPC):
***C08L 83/08*** (2006.01) ***A61B 5/256*** (2021.01) ***A61B 5/265*** (2021.01) ***C08K 3/08*** (2006.01)

(52) Cooperative Patent Classification (CPC):
**A61B 5/256; A61B 5/265; C08K 3/08; C08L 83/08**

(86) International application number:
**PCT/JP2024/030929**

(87) International publication number:
**WO 2025/074792 (10.04.2025 Gazette 2025/15)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **03.10.2023 JP 2023172057**

(71) Applicant: **NOK Corporation**
**Minato-ku**
**Tokyo 105-8585 (JP)**

(72) Inventors:
- **FURUKAWA, Tomonori**
**Fujisawa-shi, Kanagawa 2510042 (JP)**
- **HAYASHI, Taisei**
**Fujisawa-shi, Kanagawa 2510042 (JP)**

(74) Representative: **Global IP Europe Patentanwaltskanzlei**
**Pfarrstraße 14**
**80538 München (DE)**

(54) **RUBBER COMPOSITION AND BIOELECTRODE**

(57) The present invention relates to a rubber composition comprising a fluorosilicone rubber and silver particles, and a bioelectrode comprising the rubber composition.

13
10
14
11
12

FIG.2

EP 4 790 104 A1

## Description

### Technical Field

The present invention relates to a rubber composition and a bioelectrode.

### Background Art

Conventionally, rubber compositions have been used for various applications. For example, examples of the rubber composition used in the form in which the rubber composition contacts the skin of a human include bioelectrodes, rubber bands, hoses, gloves, long boots, tents, toys, and the like. Among these, there is a rubber composition imparted with electroconductivity by containing an electroconductive material.

As a particularly useful usage example of the electroconductive rubber composition, a bioelectrode that obtains an electric signal from a living body when the bioelectrode is directly contacted with the living body is known. As this bioelectrode, for example, there is a bioelectrode in which a part to contact the skin of the living body is formed with the electroconductive rubber composition. The bioelectrode formed from the electroconductive rubber composition is soft compared with an electrode made of metal, and, therefore, such a bioelectrode can obtain an electric signal from a living body in the state in which the living body is not made to feel pain. Examples of the material of the electroconductive rubber composition include a material containing vinyl methyl silicone rubber and silver particles. However, the vinyl methyl silicone rubber becomes soaked with sebum components such as squalene and oleic acid, and, therefore, there has sometimes been a case in which the sebum components derived from the living body permeate and deposit in the surface of the rubber composition, whereby the bioelectrode becomes in an insulated state, whereby it becomes impossible to obtain a biological signal.

Patent Literature 1 (Japanese Patent Application Publication No. 2018-061778) discloses an electroconductive rubber composition for measuring a biological signal comprising: a rubber component comprising at least one kind selected from the group consisting of a synthetic rubber and elastomer and having a crosslinked structure; an electroconductive metal particle; an adhesive; and a polar solvent. However, there has sometimes been a case in which the sebum components derived from the living body permeate and deposit in the surface of the bioelectrode, as described above, whereby it is impossible to use the bioelectrode in the desired state.

As exemplified by the above bioelectrode, conventional electroconductive rubber compositions have been required to stably maintain excellent electroconductivity while the sebum components derived from the skin of a living body do not permeate or deposit in the rubber composition even when the rubber composition is used while contacting the living body for a long period of time.

### Document List

#### Patent Literature

Patent Literature 1: Japanese Patent Application Publication No. 2018-061778

### Summary of Invention

#### Technical Problem

The present invention has been made in view of the above situations. That is, the present invention provides a rubber composition that stably maintains excellent electroconductivity while the sebum components derived from the skin of a living body do not permeate or deposit in the rubber composition even when the rubber composition is used while contacting the living body for a long period of time, and a bioelectrode produced by using it.

#### Solution to Problem

The constitution of the aspect of the present invention is as follows:

[1] A rubber composition comprising:

a fluorosilicone rubber; and
silver particles.

[2] The rubber composition according to the [1], wherein,

when a surface resistance value of the rubber composition after the rubber composition in a sheet form having a vertical length of 30 mm, a lateral length of 30 mm, and a thickness of 2 mm is bent 30 times as measured in accordance with JIS K7194: 1994 is defined as R1, and
a surface resistance value of the rubber composition after the rubber composition in a sheet form having a vertical length of 30 mm, a lateral length of 30 mm, and a thickness of 2 mm is bent 30 times and is then immersed in squalene at 50°C for 300 hours as measured in accordance with JIS K7194: 1994 is defined as R2,
R2/R1 is 10 or less.

[3] A bioelectrode comprising the rubber composition according to the [1] or the [2].
[4] The bioelectrode according to the [3], wherein the bioelectrode is an earpiece-shaped bioelectrode to be inserted into an external auditory canal.
[5] The bioelectrode according to the [3], wherein the bioelectrode is to be contacted with a scalp for use.

Effects of Invention

A rubber composition that stably maintains excellent electroconductivity while the sebum components derived from the skin of a living body do not permeate or deposit in the rubber composition even when the rubber composition is used while contacting the living body for a long period of time, and a bioelectrode produced by using it can be provided.

Brief Description of Drawings

[Fig. 1] A diagram schematically illustrating the earpiece-shaped bioelectrode according to an embodiment of the present invention.
[Fig. 2] A cross-sectional view schematically illustrating the bioelectrode according to an embodiment of the present invention.
[Fig. 3] A perspective view schematically illustrating the bioelectrode according to an embodiment of the present invention.
[Fig. 4] A graph showing the results of the durability test for Examples 1 to 3 and Comparative Example 1.

Description of Embodiments

The rubber composition of the present invention contains a fluorosilicone rubber and silver particles. The fluorosilicone rubber is a rubber represented by the following formula (1) obtained by copolymerizing methyltrifluoropropylsiloxane represented by the following formula (A) with methyl vinyl siloxane represented by the following formula (B), and is also expressed as FVMQ (Fluoro Vinyl Methyl Siloxane).

[Formula 1]

$$-\left[\begin{array}{c} CH_2-CH_2-CF_3 \\ | \\ Si-O \\ | \\ CH_3 \end{array}\right]_m \left[\begin{array}{c} CH=CH_2 \\ | \\ Si-O \\ | \\ CH_3 \end{array}\right]_n- \quad (1)$$

(A) (B)

The fluorosilicone rubber has a property of being difficult to become soaked with the sebum components of a living body such as squalene and oleic acid. Therefore, even when the rubber composition is contacted with the skin of a living body, the sebum components of the living body do not permeate or deposit in the rubber composition. Also, since the rubber composition contains silver particles, the rubber composition can be an electroconductive rubber composition that stably maintains a desired electroconductivity. In particular, where the rubber composition of the present invention is used as a member of the bioelectrode to be contacted with a living body, permeating and depositing of the sebum components in

the rubber composition can be inhibited for a long period of time, and, therefore, decrease in a surface resistance value can be inhibited for a long term, and the electric signal of the living body can be stably measured. The rubber composition may be a rubber composition before vulcanization or a rubber composition after vulcanization, but is preferably a rubber composition after vulcanization.

When a surface resistance value (mΩ) of the rubber composition after the rubber composition having a vertical length of 30 mm, a lateral length of 30 mm, and a height of 2 mm is bent 30 times before being immersed in squalene as measured in accordance with JIS K7194: 1994 is defined as R1, and a surface resistance value (mΩ) of the rubber composition after the rubber composition having a vertical length of 30 mm, a lateral length of 30 mm, and a height of 2 mm is bent 30 times and is then immersed in squalene at 50°C for 300 hours, and then liquid remaining on the surface of the rubber composition is wiped off as measured in accordance with JIS K7194: 1994 is defined as R2, R2/R1 is preferably 10 or less, and more preferably 2 or less. The surface resistance value R1 is preferably 3.0 mΩ or less. The surface resistance value R2 is preferably 30.0 mΩ or less, and more preferably 6 mΩ or less. The surface resistance values measured as described above are each an index showing the oil resistance of the rubber composition, and the rubber composition can have excellent oil resistance if the rubber composition has a surface resistance value of the above range. Also, the volume change rate of the rubber composition is preferably 0 to 5%, more preferably 0 to 3%, and further preferably 0 to 1%. If the rubber composition has a volume change rate of the above range, the sebum is difficult to permeate, and the rubber composition can maintain a stable electroconductivity for a long period of time. The volume change rate of the rubber composition may be measured by the method stipulated in JIS K6258: 2016 by using an apparatus of a hydrometer.

Hereinafter, each component forming the rubber composition of the present invention will be described in detail.

(Fluorosilicone rubber)

The fluorosilicone rubber is not particularly limited as long as the fluorosilicone rubber is a copolymer of methyltrifluoropropylsiloxane and methyl vinyl siloxane. A commercially available product may be used for such a fluorosilicone rubber, and examples thereof include commercially available fluorosilicone rubbers such as FE-221-U (product name), FE-251-U (product name), and FE-351-U (product name).

(Silver particles)

The characteristic of the silver particles is not particularly limited, and silver particles in the form containing an aggregated silver powder and a flaky silver powder may be used. The aggregated silver powder means silver powder in which a plurality of particulate primary particles have three-dimensionally aggregated. The flaky silver powder means scale-shaped silver powder. The average particle diameter of each of the aggregated silver powder and the flaky silver powder is not limited to a specific value, and, for example, the aggregated silver powder is preferably an aggregated silver powder having an average particle diameter in the range of 4 μm to 8 μm, and the flaky silver powder is preferably a flaky silver powder having an average particle diameter in the range of 5 μm to 15 μm. The average particle diameter of the silver particles is an average diameter calculated by performing measurement with an electron microscope photograph and then determining the arithmetic mean. The content ratios of the aggregated silver powder and the flaky silver powder are preferably (aggregated silver powder: flaky silver powder = 70: 30 to 30: 70), and, more preferably, the ratios of the aggregated silver powder and the flaky silver powder are the same.

Examples of the flaky silver powder include Product name "327077" (manufactured by Sigma-Aldrich Co. LLC), Product name "FA-D-3" (manufactured by DOWA ELECTRONICS MATERIALS CO., LTD.), Product name "FA-2-3" (manufactured by DOWA ELECTRONICS MATERIALS CO., LTD.), and the like. Among these, as the flaky silver particles, Product name "FA-2-3" (manufactured by DOWA ELECTRONICS MATERIALS CO., LTD.) is preferable. As the aggregated silver particles, for example, Product name "G-35" (manufactured by DOWA ELECTRONICS MATERIALS CO., LTD.) is preferable.

The content of the silver powder in the rubber composition may be appropriately adjusted in the range in which electroconductivity can be imparted, but is preferably in the range of 80 to 300 parts by weight, more preferably in the range of 100 to 250 parts by weight, and further preferably in the range of 150 to 200 parts by weight based on 100 parts by weight of the fluorosilicone rubber. If the content (parts by weight) of the silver powder in the rubber composition is within the above range, the rubber composition can have both excellent flexibility and excellent electroconductivity.

(Other additives)

The rubber composition of the present invention contains a fluorosilicone rubber and silver particles, but the rubber composition may further contain, as other additives, colorants such as carbon black and pigments; vulcanizing agents; processing aids such as waxes, metal soaps, and carnauba waxes; acid acceptors such as calcium hydroxide, magnesium oxide, zinc oxide, and hydrotalcite; anti-aging agents; thermoplastic resins; plasticizers; softeners; colorants;

dispersing agents; flame retardants; adhesion imparting agents; mold release agents; various metal powders; or the like.

(Method for producing rubber composition and formed article)

The method for producing the rubber composition of the present invention is not particularly limited, and the rubber composition may be produced by kneading the fluorosilicone rubber, the silver particles, and additives that are added as necessary with a kneader, a roll, or the like. After adding a vulcanizing agent as necessary to the rubber composition, the rubber composition may be formed into a desired shape by a desired method such as compression forming, transfer forming, injection forming, extrusion forming, and calender forming, and then the formed rubber composition may be vulcanized under heating at 165 to 220°C for 1 to 30 minutes to form a formed product. Thereafter, the formed product may optionally be immersed in salt water having a predetermined concentration and then the formed product may be heated at a temperature of 120 to 140°C in an autoclave to ionize the silver forming the silver particles. Also, as necessary, secondary vulcanization may be performed on the obtained formed product at 200 to 230°C for 1 to 5 hours.

The kind of the formed product is not particularly limited, and examples thereof include bioelectrodes. The bioelectrode is not particularly limited, and examples thereof include an electrode that obtains an electric signal from a living body when the bioelectrode contacts the skin of the living body. The bioelectrode containing the rubber composition produced from the rubber composition of the present invention contains the fluorosilicone rubber, and, therefore, the bioelectrode has a property of being difficult to become soaked with sebum components of the living body such as squalene and oleic acid. Therefore, at the time of measurement of the electric signal of the living body by the bioelectrode, the sebum components derived from the skin of the living body do not permeate or deposit in the bioelectrode. Also, since the bioelectrode contains the silver particles, it is possible to stably measure the accurate electric signal (for example, surface resistance value) of the living body with little electric potential fluctuation. The living body to be measured for the electric signal by the bioelectrode is not particularly limited, and examples thereof include humans, mammals other than humans, birds, and the like. The part on the living body to be contacted with the bioelectrode is not particularly limited, and examples thereof include the inside of external auditory canals, the scalp, other areas in which the skin is exposed, and the like.

(Bioelectrode)

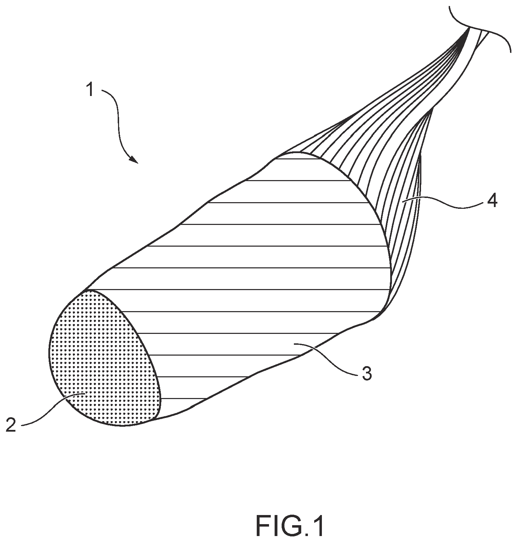

The application of the bioelectrode is not particularly limited, and the bioelectrode may be used as a medical device, a treatment device, a wearable information device, a health monitoring device, or the like in the field of medicine, nursing care, welfare, or the like. Hereinafter, the bioelectrode will be described with specific reference to embodiments of the bioelectrode. The bioelectrode shown below is merely an example, and the bioelectrode of the present invention is not limited to the bioelectrode described below. FIGS. 1 to 3 are each a diagram illustrating the bioelectrode of the present invention. Hereinafter, the bioelectrode of FIGS. 1 to 3 will be described in detail.

FIG. 1 is a schematic diagram illustrating an earpiece-shaped bioelectrode 1 of the present invention to be inserted into the external auditory canal of a living body. As illustrated in FIG. 1, the earpiece-shaped bioelectrode 1 is formed of a tip end part 2, an electroconductive layer 3 containing the rubber composition of the present invention, and a wire 4. When the earpiece-shaped bioelectrode 1 of FIG. 1 is inserted into the external auditory canal of a living body, the electroconductive layer 3 contacts the skin inside the external auditory canal. The electroconductive layer 3 obtains the electric signal of the living body through the part contacting the skin inside the external auditory canal, and the electric signal is transmitted to a measurement device, which is not illustrated, through the wire 4. Sebum components such as earwax are secreted from the skin contacting the electroconductive layer 3 inside the external auditory canal, but the electroconductive layer 3 of the earpiece-shaped bioelectrode 1 of the present invention contains the fluorosilicone rubber, and, therefore, the sebum components do not permeate or deposit in the electroconductive layer 3. Therefore, the earpiece-shaped bioelectrode 1 can stably measure the accurate electric signal of the living body with little electric potential fluctuation for a long period of time.

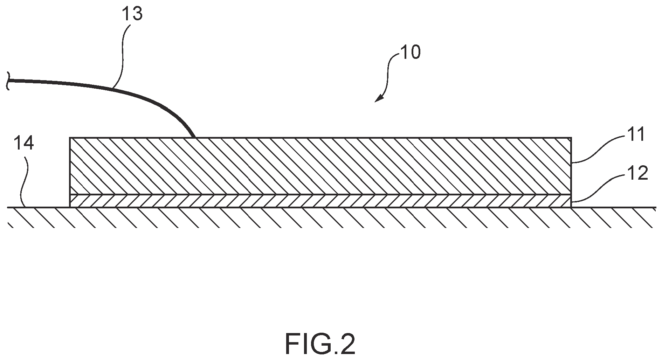

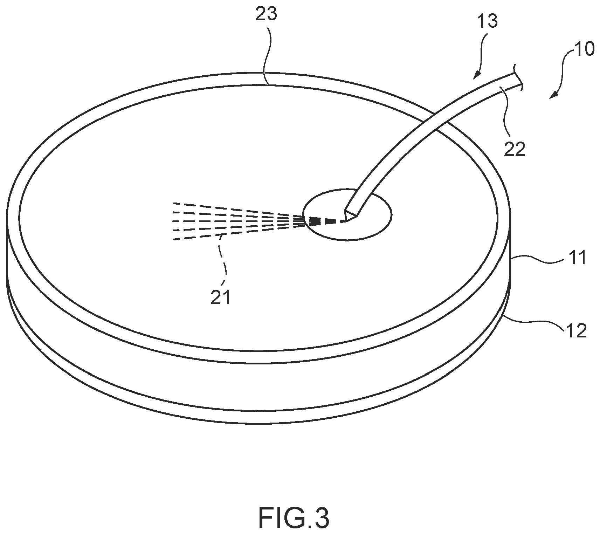

FIG. 2 is a cross-sectional view illustrating an example of the bioelectrode of the present invention, and FIG. 3 illustrates a perspective view of the bioelectrode of FIG. 2. As illustrated in FIG. 2, the bioelectrode 10 is to be directly contacted with the skin of a living body 14 for use, and the bioelectrode 10 includes an electroconductive base material 11 such as an electroconductive silicone rubber and an electroconductive layer 12 provided on the base material 11, and the electroconductive layer 12 contains the rubber composition of the present invention. A covering wire 13 is connected to the surface of the base material 11. As illustrated in FIG. 3, the covering wire 13 is formed of a core wire 21 and a covering material 22, and the core wire 21 is fixed to the surface of the base material 11 with an adhesive tape 23 or the like. When the electroconductive layer 12 of the bioelectrode 10 is contacted with the skin of the living body 14, the electric signal from the skin of the living body 14 is transmitted to a measurement device, which is not illustrated, through the electroconductive layer 12, the base material 11, and the core wire 21. Sebum components are secreted from the skin of the living body, but

the electroconductive layer 12 of the bioelectrode 10 of the present invention contains the fluorosilicone rubber, and, therefore, the sebum components do not permeate or deposit in the electroconductive layer 12. Therefore, the bioelectrode 10 can stably measure the accurate electric signal of the living body with little electric potential fluctuation. The part of the living body to be contacted with the bioelectrode 10 and the electric signal are not particularly limited, and examples of the part include the scalp, and examples of the electric signal include brain waves. Head sebum is secreted from the scalp, but the head sebum does not permeate or deposit in the electroconductive layer 12, and the bioelectrode 10 can stably measure the accurate electric signal of the living body for a long period of time.

## Examples

(Examples 1 to 3 and Comparative Example 1)

The rubbers shown in Table 1 below were each wound around an open roll and then silver particles were added, and kneading was performed with the open roll to form the rubber into a sheet form. Thereafter, the sheet was subjected to press vulcanizing at 180°C for 10 minutes and was then immersed in salt water having a concentration of 10 mass% in an autoclave to be boiled at 121°C for 60 minutes to produce a rubber sheet having a thickness of 2 mm.

[Table 1]

| | Example 1 | Example 2 | Example 3 | Comparative Example 1 |
|---|---|---|---|---|
| Material of rubber | FVMQ | FVMQ | FVMQ | VMQ |
| Silver powder ① Content of FA-2-3 (parts by weight based on 100 parts by weight of rubber) | 115 | 100 | 75 | 125 |
| Silver powder ② Content of G-35 (parts by weight based on 100 parts by weight of rubber) | 115 | 100 | 75 | 125 |
| Content of crosslinking agent (parts by weight based on 100 parts by weight of rubber) | 1 | 1 | 1 | 1 |
| Hardness (Duro-A, peak) | 70 | 56 | 49 | 70 |
| Volume resistivity (Ω ·cm) | $3.5\times10^{-4}$ | $1.3\times10^{-3}$ | $2.0\times10^{-2}$ | $7.2\times10^{-4}$ |
| Time taken for surface resistance value to reach threshold ($1\times10^{7}$mΩ) starting from initial value in durability test (time) | >300 | >300 | >300 | 24 |
| Volume change rate (%)<br>Immersion liquid: Squalene<br>Immersion condition: Room temperature, 70 hours | 0 | 0 | 0 | 10 |

The names of the materials used in each example is shown below.

FVMQ: fluorosilicone rubber; FE-221-U (product name) manufactured by Shin-Etsu Chemical Co., Ltd.

VMQ: vinyl methyl silicone rubber; LIVEO QP1-25 BASEJPN (product name) manufactured by Dow Corning Toray Co., Ltd.

Silver particles: FA-2-3 (product name) manufactured by DOWA ELECTRONICS MATERIALS CO., LTD.

Silver particles: G-35 (product name) manufactured by DOWA ELECTRONICS MATERIALS CO., LTD.

Crosslinking agent: peroxide-based crosslinking agent (2,5-dimethyl-2,5-ditertiarybutylperoxyhexane); PERHEX-A25B (product name) manufactured by Nippon Oil & Fats Co., Ltd.

Each of the sheets of the rubber compositions produced in the above manner was measured for the hardness, the volume resistivity, the durability test, and the volume change rate. The measurement method for each of the above properties is shown below.

(1) Hardness (durometer A hardness; peak) (-)

The peak hardness of the durometer A of the sheet of the rubber composition having a vertical length of 50 mm, a lateral length of 20 mm, and a thickness of 2 mm was measured by using P2-A type (apparatus name), which was an automatic rubber hardness meter, manufactured by KOBUNSHI KEIKI CO.,LTD. in accordance with JIS K6253-3: 2023.

(2) Volume resistivity ($\Omega \cdot$ cm)

The volume resistivity of the sheet of the rubber composition having a vertical length of 50 mm, a lateral length of 20 mm, and a thickness of 2 mm was measured by using Loresta-GX MCP-T700 (product name), which was a low resistivity meter, manufactured by Mitsubishi Chemical Analytech Co., Ltd. as a measurement apparatus, and ASP probe (product name) manufactured by Mitsubishi Chemical Analytech Co., Ltd. as a terminal in accordance with a four-probe method in JIS K7194: 1994.

(3) Durability test (time)

The sheet of the rubber composition having a vertical length of 30 mm, a lateral length of 30 mm, and a thickness of 2 mm was bent 30 times to apply a strain on the rubber composition. Thereafter, a surface resistance value R1 of the sheet of the rubber composition was measured by using Loresta-GP MCP-T610 (product name) manufactured by Mitsubishi Chemical Analytech Co., Ltd. and PSP probe (product name), which was a terminal, manufactured by Mitsubishi Chemical Analytech Co., Ltd in accordance with the four-probe method in JIS K7194: 1994. Thereafter, the sheet of the rubber composition was immersed in squalene at 50°C for 300 hours. While the sheet of the rubber composition was immersed in the squalene, the sheet of the rubber composition was taken out of the squalene over time and liquid on the surface of the sheet of the rubber composition was wiped off. Thereafter, a surface resistance value of the sheet of the rubber composition was measured by using Loresta-GP MCP-T610 (product name) manufactured by Mitsubishi Chemical Analytech Co., Ltd. and PSP probe (product name), which was a terminal, manufactured by Mitsubishi Chemical Analytech Co., Ltd in accordance with the four-probe method in JIS K7194: 1994. The time taken for the surface resistance value to reach $1 \times 10^7$ m$\Omega$ (threshold) during the period of 300 hours from the start of the durability test was measured. The surface resistance value of the sheet of the rubber composition after 300 hours had passed from the start of the durability test was defined as R2. Where the surface resistance value was still less than $1 \times 10^7$ m$\Omega$ (threshold) even after 300 hours had passed from the start of the durability test, the sheet of the rubber composition was evaluated as "more than 300 hours".

(4) Volume change rate (%)

The sheet of the rubber composition having a vertical length of 50 mm, a lateral length of 25 mm, and a thickness of 2 mm was immersed in squalene at room temperature (25°C) for 70 hours and was then taken out. The volumes of the sheet of the rubber composition before and after being immersed in the squalene were measured by using DSG-1 (product name), which was an automatic hydrometer, manufactured by Toyo Seiki Seisaku-sho, Ltd. The volume change rate was calculated in accordance with the following equation based on the method stipulated in JIS K6258: 2016.

Volume change rate (%) = {(Volume of sheet of rubber composition after being immersed in squalene) - (Volume of sheet of rubber composition before being immersed in squalene)} / (Volume of sheet of rubber composition before being immersed in squalene) $\times$ 100

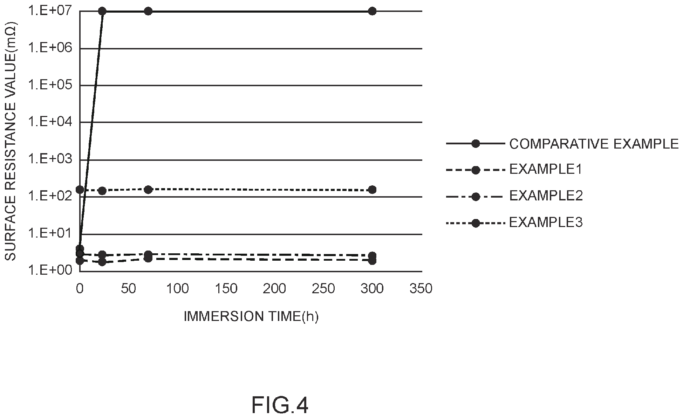

The result of the hardness, the volume resistivity, the durability test, and the volume change rate for each example as measured in the above manner is shown in above Table 1. Also, the change in the surface resistance value over time in the period of 0 to 300 hours in the durability test is shown in FIG. 4. As shown in Table 1 and FIG. 4, in each of Examples 1 and 2, the durability test maintained a low surface resistance value of 3.0 m$\Omega$ or less over the period of 0 to 300 hours and R2/R1 was 1.1 or less, and also the volume change rate had a low value of 0%. Also, in Example 3, the durability test maintained a low surface resistance value of 200 m$\Omega$ or less over the period of 0 to 300 hours and R2/R1 was 1.05 or less, and also the volume change rate had a low value of 0%. In contrast, in Comparative Example 1, the surface resistance value reached $1 \times 10^7$ m$\Omega$ (threshold) after 24 hours from the start of the durability test and R2/R1 had a value significantly exceeding 10, and also the volume change rate had a high value of 10%. As described above, it was successfully confirmed that the rubber composition of the present invention was able to stably maintain excellent electroconductivity while the sebum components did not permeate or deposit for a long period of time.

List of Reference Signs

1 earpiece-shaped bioelectrode
2 tip end part
3, 12 electroconductive layer
4 wire
10 bioelectrode
11 base material
13 covering wire
14 skin of living body
21 core wire
22 covering material
23 adhesive tape

## Claims

**1.** A rubber composition comprising:

a fluorosilicone rubber; and
silver particles.

**2.** The rubber composition according to claim 1, wherein,

when a surface resistance value of the rubber composition after the rubber composition in a sheet form having a vertical length of 30 mm, a lateral length of 30 mm, and a thickness of 2 mm is bent 30 times as measured in accordance with JIS K7194: 1994 is defined as R1, and
a surface resistance value of the rubber composition after the rubber composition in a sheet form having a vertical length of 30 mm, a lateral length of 30 mm, and a thickness of 2 mm is bent 30 times and is then immersed in squalene at 50°C for 300 hours as measured in accordance with JIS K7194: 1994 is defined as R2,
R2/R1 is 10 or less.

**3.** A bioelectrode comprising the rubber composition according to claim 1 or 2.

**4.** The bioelectrode according to claim 3, wherein the bioelectrode is an earpiece-shaped bioelectrode to be inserted into an external auditory canal.

**5.** The bioelectrode according to claim 3, wherein the bioelectrode is to be contacted with a scalp for use.

1
4
3
2

FIG.1

13
10
11
14
12

FIG.2

23
13
10
22
21
11
12

FIG.3

1.E+07
1.E+06
1.E+05
1.E+04
1.E+03
1.E+02
1.E+01
1.E+00
SURFACE RESISTANCE VALUE(mΩ)
0
50
100
150
200
250
300
350
IMMERSION TIME(h)
COMPARATIVE EXAMPLE
EXAMPLE1
EXAMPLE2
EXAMPLE3

FIG.4

**INTERNATIONAL SEARCH REPORT**

International application No. **PCT/JP2024/030929**

**A. CLASSIFICATION OF SUBJECT MATTER**

***C08L 83/08***(2006.01)i; ***A61B 5/256***(2021.01)i; ***A61B 5/265***(2021.01)i; ***C08K 3/08***(2006.01)i
FI: C08L83/08; A61B5/256 100; A61B5/256 130; A61B5/265; C08K3/08

According to International Patent Classification (IPC) or to both national classification and IPC

**B. FIELDS SEARCHED**

Minimum documentation searched (classification system followed by classification symbols)

C08L1/00-101/16; C08K3/00-13/08; A61B5/00-5/398

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | CN 107325748 A (AECC BEIJING INSTITUTE OF AERONAUTICAL MATERIALS) 07 November 2017 (2017-11-07) claims, examples section (e.g. "6" in table 1), etc. | 1-2 |
| Y | | 3-5 |
| Y | JP 2021-44066 A (FUJI XEROX CO., LTD.) 18 March 2021 (2021-03-18) claims, paragraphs [0027], [0057], examples section, etc. | 3-5 |
| A | WO 2018/230445 A1 (NOK CORPORATION) 20 December 2018 (2018-12-20) | 1-5 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 November 2024** | **19 November 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/030929**

| Patent document cited in search report | Publication date (day/month/year) | Patent family member(s) | Publication date (day/month/year) |
|---|---|---|---|
| CN 107325748 A | 07 November 2017 | (Family: none) | |
| JP 2021-44066 A | 18 March 2021 | US 2021/0070943 A1<br>claims, paragraphs [0040], [0093], examples section, etc.<br>CN 112450936 A | |
| WO 2018/230445 A1 | 20 December 2018 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2018061778 A